Europäisches Patentamt

European Patent Office

Office européen des brevets

(19)

(11) Veröffentlichungsnummer : **0 414 635 B1**

(12) **EUROPÄISCHE PATENTSCHRIFT**

(45) Veröffentlichungstag der Patentschrift :
**25.11.92 Patentblatt 92/48**

(51) Int. Cl.$^5$ : **G04B 19/26,** G06C 3/00

(21) Anmeldenummer : **90810604.0**

(22) Anmeldetag : **13.08.90**

(54) **Uhr mit einer Einrichtung zur Geschlechtsplanung nach der Zeitwahlmethode.**

(30) Priorität : **21.08.89 CH 3024/89**

(43) Veröffentlichungstag der Anmeldung :
**27.02.91 Patentblatt 91/09**

(45) Bekanntmachung des Hinweises auf die
Patenterteilung :
**25.11.92 Patentblatt 92/48**

(84) Benannte Vertragsstaaten :
**AT BE CH DE DK ES FR GB GR IT LI LU NL SE**

(56) Entgegenhaltungen :
**BE-A- 729 841
CH-A- 294 405
CH-A- 373 700
DE-A- 2 949 087**

(73) Patentinhaber : **B-LINE AG
Rain 26
CH-3373 Heimenhausen (CH)**

(72) Erfinder : **Riesen, Heinz
Kirchstrasse 22
CH-4227 Büsserach (CH)**

(74) Vertreter : **Eschmann, Heinz et al
A. Braun, Braun, Héritier, Eschmann AG
Patentanwälte Holbeinstrasse 36-38
CH-4051 Basel (CH)**

EP 0 414 635 B1

**Beschreibung**

Die Erfindung bezieht sich auf eine Uhr mit einer Einrichtung nach dem Oberbegriff des Patentanspruchs 1.

Die dort angesprochene Zeitwahlmethode ist u.a. in dem Buch von Dr. Otfried Hatzold, "Wunschkind Sohn oder Tochter" München 1985 (4. Aufl.) sowie dem Forschungsbericht JSBN Nr. 3-89 162/003/9 des gleichnamigen Autors dargestellt. Eine Berechnungstafel zur Geburtenplanung nach dieser Methode ist aus DE 29 49 087 A1 bekannt.

Bei der bekannten Berechnung wird von der Erkenntnis Gebrauch gemacht, daß der Zeitpunkt der Zeugung innerhalb des Zyklus aus folgenden Gründen geschlechtsbestimmend ist: Die weibliche Eizelle ist geschlechtsneutral, und die männlichbestimmenden Samenfäden wandern schneller zum Ei als weiblichbestimmende, letztere bleiben aber länger, nämlich etwa zwei Tage lang, befruchtungsfähig. Bei einem Geschlechtsverkehr zwei Tage vor dem Eisprung kann daher mit hoher Wahrscheinlichkeit ein Mädchen erwartet werden, da die männlichbestimmenden Samenfäden diesen Zeitraum in der Regel nicht überleben und lediglich die weiblichbestimmenden zur Befruchtung gelangen. Erfolgt hingegen der Geschlechtsverkehr am Tag des Eisprungs, ist die Wahrscheinlichkeit für die Befruchtung mit einem männlichbestimmenden Samenfaden hoch, da diese aufgrund ihrer schnelleren Beweglichkeit zuerst auf das Ei treffen.

Die Anwendung dieser Erkenntnisse hat im Rahmen eines in dem obengenannten Buch beschriebenen Forschungsprojekts eine Erfolgsquote von etwa 90% ergeben. Um jedoch eine derart hohe Erfolgsquote zu erzielen, ist es natürlich Voraussetzung, den Tag des Eisprungs in jedem neuen Zyklus mit großer Wahrscheinlichkeit bestimmen zu können. Hier kann ein Aufzeichnen möglichst vieler Zyklusverläufe aus der Vergangenheit, insbesondere während der letzten 12 Monate, eine gewisse Sicherheit bezüglich zu erwartender Schwankungen geben, da der Tag des zurückliegenden Eisprungs vom ersten Tag jeder neuen Menstruation mit hoher Regelmäßigkeit einen Abstand von fünfzehn Tagen besitzt. Die mit dem Tag des Menstruationsbeginns feststellbaren Zyklusschwankungen gestatten somit einen sehr zuverlässigen Rückschluß auf die entsprechende Verschiebung des Tages, an dem der letzte Eisprung stattfand. Je geringer die festgestellten Zyklusschwankungen sind, umso größer ist die Voraussagewahrscheinlichkeit. Eine in der Vergangenheit auch in Fachkreisen nicht seltene Berechnungsunsicherheit läßt sich heute durch eine sorgfältige Anwendung der obengenannten Berechnungstafel ausschließen.

Es sind bereits Uhren bekannt, z.B. BE-A-72 98 41, die ein Ablesen der Daten eines Menstruationszyklus unter Berücksichtigung von Zyklusschwankungen gestatten. Auch ist es bekannt, bei einer Uhr unterschiedliche Farb- oder Bildzeichen zur Sichtbarmachung verschiedener Zeiträume innerhalb eines Menstruationszyklus zu verwenden (CH-A-294 405). Für eine Geschlechtsplanung eignen sich diese bekannten Uhren jedoch nicht.

Es ist die Aufgabe der Erfindung, eine Uhr der eingangs genannten Art zu schaffen, die innnerhalb eines bestimmten Zyklusschwankungsbereichs einen jederzeitigen Überblick sowohl über die fruchtbaren und unfruchtbaren Tage als auch über den Zeitraum des Eisprungs und die Empfängniswahrscheinlichkeit für Mädchen oder Jungen gibt und von der Benutzerin keinerlei Berechnungen verlangt.

Diese Aufgabe wird durch die im Kennzeichen des Patentanspruchs 1 definierten Merkmale gelöst. Weitere Ausgestaltungen von Ausführungsarten des Erfindungsgegenstandes ergeben sich aus den abhängigen Patentansprüchen.

Bei der erfindungsgemäßen Uhr werden die obengenannten Rechenfehler dadurch mit Sicherheit ausgeschlossen, daß die Positionsfelder für die jeweiligen Zeugungstage nach dem ersten Tag der neuen Menstruation und nicht nach der Zykluslänge nummeriert sind.

Die Uhr nach der Erfindung berücksichtigt monatliche Zyklusschwankungen von vier Tagen. Die entsprechenden Zykluslängen betragen zwischen 27 und 30 Tagen; diese werden durch die Positionsfelder für den 28ten, den 29ten, den 30ten und den 31ten Tag repräsentiert.

Eine leicht überschaubare Anordnung der Positionsfelder wird bei der erfindungsgemäßen Uhr dadurch erreicht, daß die Positionsfelder aller "Mädchen"-tage entsprechend der Zykluslänge getrennt bzw. abgesetzt von denen aller "Jungen"-tage entsprechend der Zyklusdauer markiert sind. Diese Markierungen sind vorteilhafterweise in konzentrisch zueinander versetzten Kreissegmenten angeordnet.

Da nach einer Ausführungsart der Erfindung die Positionsfelder des Zyklusschwankungs-Anzeigebereichs die letzten vier Tage vor der Ausgangsposition besetzen und mit den entsprechend ansteigenden Positionsziffern kenntlich gemacht sind, ist es ohne weiteres möglich, am ersten Tag des neuen Zyklus das betreffende Positionsfeld zur Erfassung der Zykluslänge direkt abzulesen, die Feldziffer zu notieren und die Anzeige wieder auf ihre Ausgangsposition zurückzustellen.

Weitere Merkmale und Vorteile der Erfindung ergeben sich aus der nachstehenden Beschreibung von Ausführungsbeispielen anhand von Zeichnungen. Es zeigen:

Fig. 1 eine schematische Darstellung einer nach den Merkmalen der Erfindung gestalteten Uhr mit einer Zykluszeiger-Anzeige,

Fig. 2 eine schematische Darstellung von Antriebsdetails einer Uhr gemäß Fig. 1,

Fig. 3 zwei Darstellungen von Uhren gemäß Fig. 1 mit einer im (A) und einer gegen den (B) Uhrzeigersinn umlaufenden Zykluszeiger-Anzeige und

Fig. 4 eine schematische Darstellung einer weiteren Ausführungsform einer nach den Merkmalen der Erfindung gestalteten Uhr mit einer Datumsfenster-Anzeige.

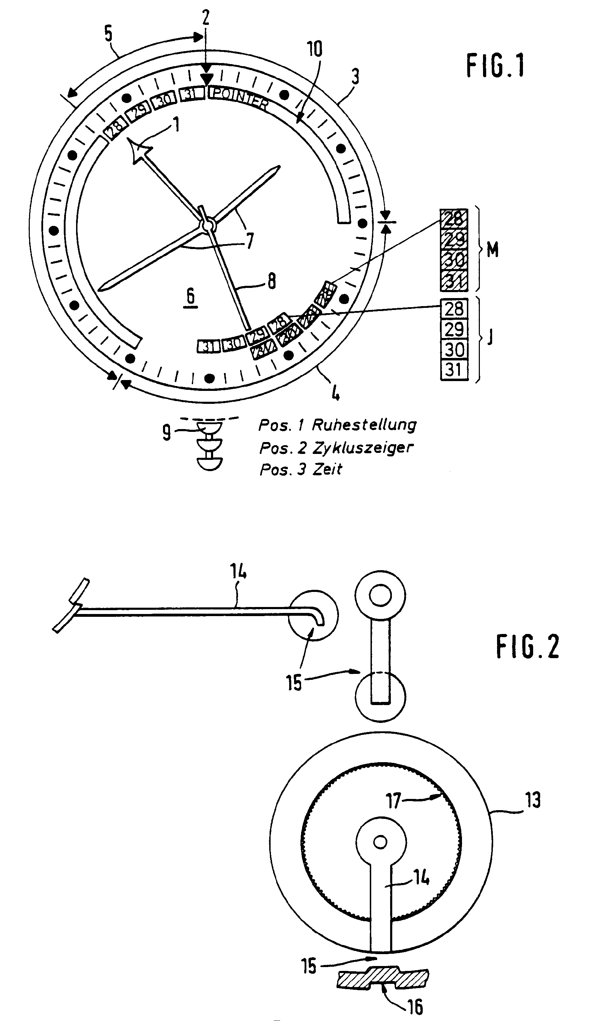

Die Uhr gemäß Fig. 1 besitzt ein Zifferblatt 6, ein Paar Minuten- und Stundenzeiger 7, einen Sekundenzeiger 8 und ein Anzeigeelement 1 in Form eines Zykluszeigers. Hinter der sichtbaren Zifferblattfläche befindet sich ein genaugängiges Zeitwerk herkömmlicher Bauart. Das Zifferblatt 6 ist mit einer üblichen Stunden- und Minuteneinteilung versehen, wobei die 12-Uhr Position mit einem Dreiecksymbol besonders gekennzeichnet ist. Eine Einstellkrone 9 befindet sich gegenüber der 6-Uhr Position, und ist zwischen den drei dargestellten Positonen verschiebbar, wobei Pos. 1 die Ruhestellung ist, Pos. 2 zur Ein- und Verstellung des Zykluszeigers 1 dient und Pos. 3 zur Zeiteinstellung.

Konzentrisch innerhalb der Stunden- und Minuteneinteilung befindet sich ein ringförmiger Anzeigebereich 10, welcher in folgende Abschnitte unterteilt ist: einen Abschnitt 3, der die unfruchtbaren Tage eines Zyklus markiert, einen Abschnitt 4, der die fruchtbaren Tage angibt und einen Abschnitt 5 zur Anzeige eines Zyklusschwankungsbereichs. Ausgehend von einer mit der 12-Uhr Position der Uhr übereinstimmenden Ausgangsposition 2 ist der Anzeigebereich 10 in 31 Tagespositionsfelder unterteilt, welche der Zykluszeiger 1, wie ein Datumszeiger über einen Monat, in 30 Schritten durchläuft, bzw. mit dem 31ten Schritt wieder auf die Ausgangsposition 2 springt. Bei der dargestellten Ausführungsform werden Zyklusschwankungen von vier Tagen berücksichtigt (entspr. Zykluslängen von 27 bis 30 Tagen).

Innerhalb des Abschnitts 4, der die fruchtbaren Tage angibt, befinden sich konzentrisch zueinander versetzt zwei Abschnittsegmente M und J. Jedes Segment ist in vier mit 28, 29, 30 und 31 bezeichnete Tagespositionsfelder, entsprechend dem Zykluschwankungsbereich, unterteilt, und beide Segmente überlappen sich um zwei Tagespositionen. Die mit M gekennzeichneten Tage sind die "Mädchen"-tage entsprechend der Zyklusdauer, die mit J bezeichnen die "Jungen"-tage. Zwischen diesen Abschnittsegmenten M und J und den Enden des Abschnitts 3 befinden sich jeweils zwei Tagespositionen als Sicherheitsabstand. In der praktischen Ausführung können die "Mädchen"-tage rosa und die "Jungen"-tage blau gefärbt werden.

Für die Anwendung der Uhr zur Geschlechtsbestimmung ist die Kenntnis des individuellen Zyklusverlaufs entscheidend. Wenn beispielsweise während der letzten 12 Zyklen (ohne Pilleneinnahme) entsprechende Aufzeichnungen gemacht wurden, die zeigen, daß ein Zyklusschwankungsbereich innerhalb des obengenannten Schwankungsbereichs vorliegt, kann die Uhr mit Beginn der nächsten Menstruation eingesetzt werden. Hierzu wird lediglich am ersten Tag des neuen Zyklus der Zykluszeiger 1 auf die Ausgangsposition 2 gestellt. Erreicht der Zeiger beispielsweise bei einer aus den Aufzeichnungen hervorgehenden Zyklusdauer von 28 Tagespositionen im Abschnitt 4 das Feld M mit der Ziffer 28, so besteht an dem betreffenden Tag eine erhöhte Wahrscheinlichkeit für die Zeugung eines Mädchens. Entsprechendes gilt für die Zyklusdauer nach den anderen Ziffern sowie für die Tagespositionen bei J für Jungen.

Wenn der Zeiger auf seinem weiteren Weg wieder den Zyklusschwankungsbereich 5 erreicht und die Menstruation zum Beispiel an der Tagesposition 30 dieses Bereichs beginnen sollte, so ist diese Positionsnummer zu notieren und der Zykluszeiger 1 wieder auf seine Ausgangsposition 2 zu stellen.

Bei Zyklusschwankungen innerhalb des angegebenen Bereichs ist bei einem Mädchenwunsch als Zeugungstag dasjenige Feld M auszuwählen, das der niedrigsten notierten Positionsnummer entspricht, für einen Jungenwunsch hingegen das Feld J mit der höchsten notierten Positionsnummer. Ferner ist es wichtig, daß innerhalb des Abschnitts 4 für die fruchtbaren Tage bei Mädchenwunsch nach dem Zeugungstag und bei Jungenwunsch vor dem Zeugungstag kein ungeschützter Verkehr stattfinden darf.

Fig. 2 zeigt eine Ringscheibe 13, welche das Aussehen einer Datumsanzeigescheibe besitzt. Am Innendurchmesser dieser Scheibe ist ein Antriebszahnkranz 17 üblicher Bauart vorgesehen, über welche die Scheibe 13 vom nicht dargestellten Zeitwerk tageweise weitergeschaltet werden kann. Um diese Antriebsbewegung auf den Zykluszeiger 1 zu übertragen, ist ein Antriebsfinger 14 vorgesehen, der mit seinem abgeknickt ausgeführten Ende 15 in eine Randkerbe 16 der Scheibe eingreift. Wenn die Scheibe 13, mittels des Positionsknopfes oder durch Zeitwerkweiterschaltung gedreht wird, erfolgt eine entsprechende Schwenkung des Fingers 14, der seinerseits den mit ihm verbundenen Ziffernzeiger 1 weiterbewegt. Mittels dieser einfachen Antriebsart ist die erfindungsgemäße Einrichtung ohne größere Produktionsumstellungen an herkömmliche Uhren zu adaptieren.

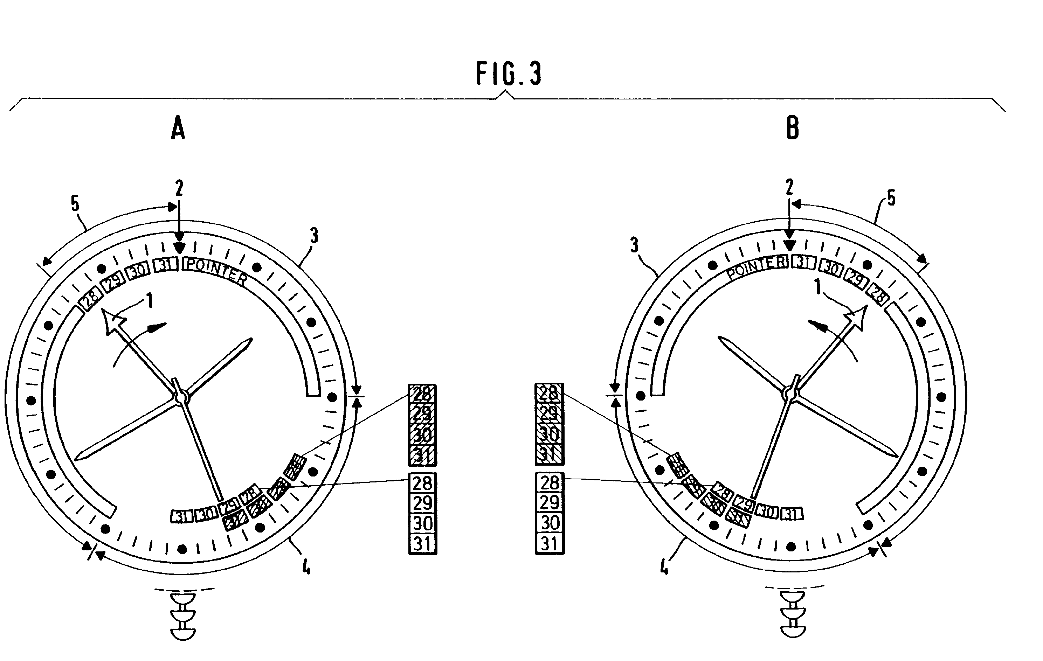

Fig. 3 zeigt zwei spiegelsymmetrisch ausgeführte Anzeigeabschnitte 3, 4 und 5 für einen rechtsdrehend (A) und einen linksdrehend (B) ausgeführten Zykluszeiger.

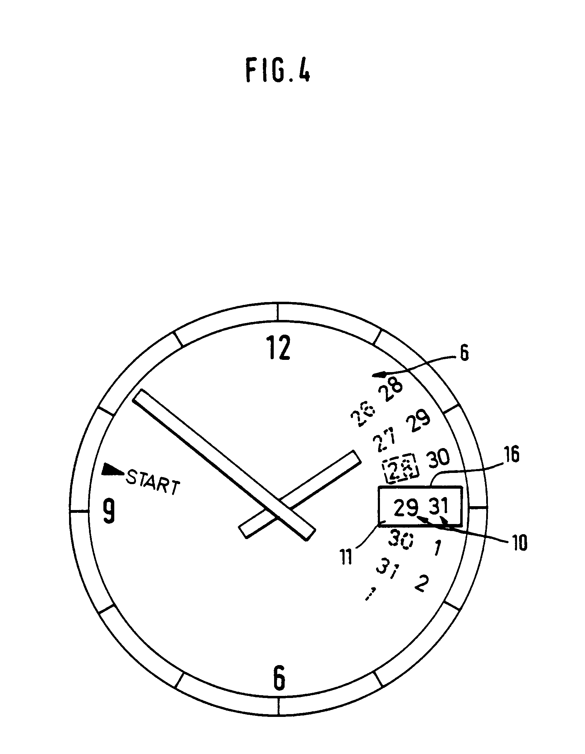

Bei Fig. 4 sind die in den Fig. 1 und 3 auf dem Zifferblatt 6 vorgesehenen Abschnitte 3, 4, 5, J und M auf einer Datumsanzeigescheibe 15 vorgesehen. Anstelle des Zykluszeigers ist ein Fenster 16 vorgesehen, das die jeweilige Positionsziffer sichtbar werden läßt.

## Patentansprüche

1. Uhr mit einer Einrichtung zur Geschlechtsplanung nach der Zeitwahlmethode, mit einem vom Zeitwerk angetriebenen, einstellbaren Anzeigeelement (1, 11), das, ähnlich einer Datumsanzeige, jeweils eine innerhalb eines Anzeigebereichs (10) vorgegebene Tagesposition bezeichnet, wobei der Anzeigebereich, bezogen auf eine Anzeige-Ausgangsposition (2), unterschiedlich markierte Anzeigeabschnitte (3, 4 und 5) zur Kenntlichmachung der fruchtbaren und unfruchtbaren Tage eines Zyklus sowie eines begrenzten Zyklusschwankungsbereichs umfaßt, dadurch **gekennzeichnet, daß** innerhalb des Anzeigeabschnitts für die fruchtbaren Tage (4) deutlich unterscheidbar markierte Tagespositionsfelder (M und J) in - nach der Zeitwahlmethode festgelegten - unterschiedlichen Tagesabständen von der Ausgangsposition (2) vorgesehen sind, welche, für Zykluslängen von 27 bis 30 Tagen, zur Angabe der Wahrscheinlichkeitstage für die Zeugung eines Jungen oder eines Mädchens dienen, wobei für die stets nach dem ersten Tag eines neuen Zyklus erfolgende Zählung und Anzeige der Wahrscheinlichkeitstage das Anzeigeelement (1, 11) auf die Ausgangsposition (2) einstellbar ist.

2. Uhr nach Anspruch 1, dadurch **gekennzeichnet, daß** innerhalb der Anzeigeabschnitte (3, 4 und 5) die Tagespositionsfelder für die "Mädchen"-tage (M) entsprechend der jeweiligen Zykluslänge getrennt bzw. abgesetzt von denen der "Jungen"-tage (J), ebenfalls entsprechend der Zyklusdauer, markiert sind.

3. Uhr nach Anspruch 2, dadurch **gekennzeichnet, daß** die besagten Tagespositionsfelder (M und J) in Form konzentrisch zueinander versetzter, verschiedenfarbiger Kreissegmentbögen angeordnet sind.

4. Uhr nach einem der Ansprüche 1 bis 3, dadurch **gekennzeichnet, daß** die Tagespositionsfelder des Zyklusschwankungs-Anzeigebereichs (5) die letzten vier Tagesfelder vor der Ausgangsposition (2) besetzen und mit entsprechend der Antriebsrichtung des Anzeigeelements (1, 11) ansteigenden Positionsziffern (28, 29, 30 und 31) kenntlich gemacht sind.

5. Uhr nach einem der Ansprüche 1 bis 4, mit einem konzentrisch-peripheren Antrieb für eine Datumsanzeige, dadurch **gekennzeichnet, daß**

ein konzentrisch-zentraler Antrieb (14, 15) des Zykluszeigers (1) vom konzentrisch-peripheren Antrieb (13, 16 und 17) für die Datumsanzeige abgeleitet ist.

6. Uhr nach einem der Ansprüche 1 bis 4, mit einer Datumsanzeigescheibe und einem zugehörigen Antrieb, deren konzentrisch auf der Anzeigescheibe plazierte Datumsziffern jeweils durch ein Fenster (16) im Zifferblatt (6) sichtbar sind, dadurch **gekennzeichnet, daß** die unterschiedlichen Anzeigeabschnitte (3, 4 und 5) durch verschiedene Farb- und/oder Gestaltgebung der Datumsziffern und/oder ihres Hintergrundes voneinander abgehoben sind.

7. Uhr nach einem der Ansprüche 1 bis 6, dadurch **gekennzeichnet, daß** das Anzeigeelement (1, 11) entweder im Uhrzeigersinn oder in der Gegenrichtung angetrieben wird und daß der Anzeigebereich (10) dementsprechend spiegelsymmetrisch bezüglich der Ausgangsposition (2) ausgebildet ist.

## Claims

1. Clock comprising a device for planning the sex of an unborn child by the preset time method, comprising an adjustable indicator element (1, 11), driven by the clock mechanism, which indicates similarly to a date indicator a predetermined day position within an indicating range (10), wherein the indicating range, referred to an indication starting position (2), comprises differently marked indication sections (3, 4 and 5) for identifying the fertile and infertile days of a cycle and also a limited cycle fluctuation range, characterized in that, within the indication section for the fertile days (4), clearly distinguishable day position fields (M and J) are provided at different day distances - determined by the preset time method - from the starting position (2), which (different day position fields) serve for informing about the probability dates for the conception of a boy or of a girl for cycle lengths of from 27 to 30 days, the indicator element (1, 11) being resettable to the starting position (2) for the counting and indication of the probability days, which is always performed from the first day of a new cycle.

2. Clock according to Claim 1, characterized in that, within the indication sections (3, 4 and 5), the day position fields for the "girl" days (M) corresponding to the particular cycle length are marked separately and/or offset from the day position fields for the "boy" days (J), also corresponding to the cycle length.

3. Clock according to Claim 2, characterized in that the afore-mentioned day position fields (M and J) are arranged in the form of mutually concentric, offset, differently coloured circular arc segments.

4. Clock according to one of Claims 1 to 3, characterized in that the day position fields of the cycle fluctuation indication section (5) occupy the last four day fields before the starting position (2) and are identified by position numbers (28, 29, 30 and 31) which increase in the direction of driving of the indicator element (1, 11).

5. Clock according to one of Claims 1 to 4, comprising a concentric-peripheral drive for a date indication, characterized in that a concentric-central drive (14, 15) of the cycle indicator (1) is derived from the concentric-peripheral drive (13, 16 and 17) for the date indication.

6. Clock according to one of Claims 1 to 4, comprising a date indication disc and an associated drive, the date numbers of which, situated concentrically on the indication disc, are visible each time through a window (16) in the dial (6), characterized in that the different indication sections (3, 4 and 5) are distinguished from one another by different colouration and/or shape of the date numbers and/or of their background.

7. Clock according to one of Claims 1 to 6, characterized in that the indicator element (1, 11) is driven either clockwise or counter-clockwise and that the indicating range (10) is accordingly constructed mirror-symmetrical with respect to the starting position (2).

**Revendications**

1. Montre équipée d'un dispositif permettant de choisir le sexe d'un enfant d'après la méthode de la sélection du moment de fécondation, comportant un élément d'affichage (1, 11) réglable, entraîné par le mouvement d'horlogerie, qui, comme un affichage de quantième, désigne chaque fois une position de jour prédéfinie dans un domaine d'affichage (10), le domaine d'affichage, comportant, rapportés à une position d'affichage de départ (2), des segments d'affichage marqués de manière différente (3, 4 et 5) pour identifier les jours de fertilité et de non-fertilité d'un cycle, ainsi qu'un domaine de fluctuation du cycle, caractérisée en ce que, dans le segment d'affichage pour les jours de fertilité (4) sont prévues, des plages de positions de jours (F et G) marquées de manière nettement discernable qui sont espacées d'un nombre de jours établi d'après la méthode de sélection du moment de fécondation et compté à partir de la position de départ, ces plages de positions de jours, pour des longueurs de cycle de 27 à 30 jours, servant à indiquer les jours de probabilité pour la conception d'un garçon ou d'une fille, l'élément d'affichage (1, 11) pouvant être réglé sur la position de départ (2) pour le comptage et l'affichage des jours de probabilité qui s'effectuent chaque fois après le premier jour d'un nouveau cycle.

2. Montre suivant la revendication 1, caractérisée en ce que, dans les segments d'affichage (3, 4 et 5), les plages de positions de jours pour les jours des "filles" (F) sont marquées, en fonction de la longueur du cycle en question, séparément ou avec un décalage par rapport à celles des jours des "garçons" (G) qui sont également fonction de la longueur du cycle.

3. Montre suivant la revendication 2, caractérisée en ce que lesdites plages de positions de jours (F et G) sont disposées sous la forme de segments d'arc de cercle de couleurs différentes décalés concentriquement l'un de l'autre.

4. Montre suivant l'une quelconque des revendications 1 à 3, caractérisée en ce que les plages de positions de jours du domaine d'affichage de fluctuation du cycle (5) occupent les quatre dernières plages de jours avant la position de départ (2) et sont identifiées par des chiffres de position (28, 29, 30 et 31) croissant selon le sens d'entraînement de l'élément d'affichage (1, 11).

5. Montre suivant l'une quelconque des revendications 1 à 4, équipée d'un entraînement périphérique concentrique pour un affichage de quantième, caractérisée en ce qu'un entraînement central concentrique (14, 15) de l'aiguille de cycle (1) est dérivé de l'entraînement périphérique concentrique (13, 16 et 17) prévu pour l'affichage de quantième.

6. Montre suivant l'une quelconque des revendications 1 à 4, comportant un disque afficheur de quantième et un entraînement associé, dont les chiffres de quantième placés concentriquement sur le disque d'affichage sont chaque fois visibles à travers un guichet (16) dans le cadran (6), caractérisée en ce que les différents segments d'affichage (3, 4 et 5) tranchent les uns par rapport aux autres par des couleurs et/ou des configurations différentes des chiffres de quantième et/ou par leur fond.

7. Montre suivant l'une quelconque des revendications 1 à 6, caractérisée en ce que l'élément d'affichage (1, 11) est entraîné soit dans le sens des aiguilles de la montre, soit dans le sens inverse et que le domaine d'affichage (10) est, en conséquence, d'une configuration énantiomorphe par rapport à la position de départ (2).

FIG.1

28 29 30 31 POINTER

M
28
29
30
31

J
28
29
30
31

6

7

8

Pos. 1 Ruhestellung
Pos. 2 Zykluszeiger
Pos. 3 Zeit

FIG.2

14

15

13

17

14

15

16

# FIG.3

A

B

EP 0 414 635 B1

# FIG.4